(19) Europäisches Patentamt
European Patent Office
Office européen des brevets

(11) Publication number: 0 138 983 B1

(12) **EUROPEAN PATENT SPECIFICATION**

(45) Date of publication of patent specification: **10.05.89**

(51) Int. Cl.[4]: **C 07 D 213/89**, A 01 N 47/36

(21) Application number: **84901649.8**

(22) Date of filing: **20.03.84**

(86) International application number: **PCT/US84/00424**

(87) International publication number: **WO 84/03884 11.10.84 Gazette 84/24**

# (54) N-PHENYL-N'-(PYRIDINYL-N-OXIDE)UREA PLANT REGULATORS.

(30) Priority: **29.03.83 US 480055**
**06.03.84 US 586574**

(43) Date of publication of application:
**02.05.85 Bulletin 85/18**

(45) Publication of the grant of the patent:
**10.05.89 Bulletin 89/19**

(84) Designated Contracting States:
**DE FR GB**

(56) References cited:
**EP-A-0 067 511**
**GB-A-1 147 438**
**US-A-3 547 935**
**US-A-3 931 201**
**US-A-3 962 265**
**US-A-4 003 733**
**US-A-4 193 788**
**US-A-4 279 639**
**US-A-4 308 054**

**Shokubutsu no Kagaku Chosetsu 17 (1982), pages 27-43 (K)**

**The file contains technical information submitted after the application was filed and not included in this specification**

(73) Proprietor: **FMC Corporation**
**2000 Market Street**
**Philadelphia Pennsylvania 19103 (US)**

(72) Inventor: **STICKER, Robert, Earl**
**4252 Freeman Road**
**Middleport, NY 14105 (US)**
Inventor: **GREEN, Christine, Mary**
**43 Meadow Run Drive**
**Skillman, NJ 08558 (US)**
Inventor: **HENRIE, Robert, Neil, II**
**44 Princeton Arms North I**
**Cranbury, NJ 08512 (US)**

(74) Representative: **Vossius & Partner**
**Siebertstrasse 4 P.O. Box 86 07 67**
**D-8000 München 86 (DE)**

(56) References cited:
**Proc.Roy.Soc., London, publ 1981, Bruce and Zwar, Cytokinin activity of some substituted ureas and thioureas, pp. 245-265**
**Chem.Pharm.Bull.Japan, publ. 1981, Okamoto et al., vol. 29, no. 12, 4-Pyridylureas are surprisingly Potent Cytokinins. The Structure-Activity Relationship., pp. 3748-3750**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

**Phytochemistry, England, pub. 1978, Takahashi et al., vol. 17, Cytokinin Activity of N-Phenyl-N'-(4-Pyridyl) Urea Derivatives, pp. 1201-1207**

**Proceedings, Intl. Colloquium of Centre National de la Recherche Scientifique, Gif-sur-Yvette (France), pub. 1981, Isogai, pp. 118-128**

## Description

The present invention relates to N-phenyl-N'-(pyridinyl-N-oxide)ureas as plant regulators for agricultural crops such as wheat, corn, cotton, soybean and the like, to novel compositions thereof, and to a method for regulating growth and development of agricultural crops.

Substantial research efforts have been devoted to finding new chemical compounds which exhibit cytokinin-like hormonal activity in agricultural crops. Such activity can beneficially affect the course of plant development in many ways. For example it can accelerate plant growth, modify plant growth or development in such a way as to increase yield, insure flowering or fruiting at a desired period in time, prevent or promote abscission, i.e., the falling of fruit or flowers from plants, increase the weight of leaves or stalks, retard senescence of the plant, and/or exhibit various other properties desirable in the growth and development of various plants. Compounds which perform such functions are commonly known as "plant regulators" and are hereinafter referred to by that term.

Typical compounds which have exhibited plant regulator activity include 6-benzyladenine, kinetin, and 4-pyridylphenylurea. US—A—4,193,788 discloses the use of certain N-(2-chloro-4-pyridyl)ureas as plant regulators. Likewise US—A—4,279,639 and US—A—4,308,054 disclose various other N-(2- and/or 6-substituted-4-pyridyl)-N'-(optionally substituted phenyl)ureas as plant regulators.

Substituted pyridinyloxy(thio)phenyl alkenyl- and alkynyl urea compounds and N-oxide derivatives which are useful as herbicides are disclosed in US—A—3,962,265. A method of inducing tillering using pyridine derivatives and agricultural compositions containing them are known from EP—A—67,511. US—A—4,003,733 describes substituted pyridinyloxy(thio)phenyl-acetamides, -ureas and urea derivatives and N-oxide derivatives thereof which are useful as herbicides. Halophenylpyridine derivatives and their use as herbicides are disclosed in GB—A—1,147,438. In US—A—3,547,935 nitro-4-pyridinols, N-oxides thereof and derivatives thereof are described.

Y. Isogai reports in Shokubutsu no Kagaku Chosetsu (Chemical Regulation in Plants), Vol. 17 (1982), pp. 27—43, on shoot formation in response to cytokinins in cultured tobacco callus and some problems associated with the phenomenon. In Table 10 of this article the activities of N-phenyl-N'-(4-pyridinyl-N-oxide) urea, N'-(2-chloro-4-pyridinyl-N-oxide)-N-phenylurea, N'-(2-chloro-4-pyridinyl-N-oxide)-N-(4-methylphenyl) urea, and N'-(2-chloro-4-pyridinyl-N-oxide)-N-(3-fluorophenyl) urea are described.

In accordance with the foregoing, the present invention comprises plant regulators of the formula

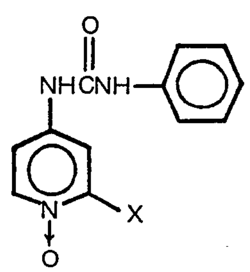

in which X is $C_{1-2}$ alkyl or $C_{1-2}$ alkylthio.

The composition aspect of the present invention provides a composition containing a plant regulating amount of the compounds described herein in admixture with an agriculturally acceptable carrier, diluent, extender or adjuvant.

In the composition aspect of this invention, the plant regulator compounds of this invention, like most agricultural chemicals, are generally not applied full strength, but are formulated with agriculturally acceptable carriers normally employed for facilitating the dispersion of active ingredients, various additives, and optionally with other active ingredients, recognizing that the formulation and mode of application of the active component may affect the activity of the material. The present compounds may be applied, for example, as powders or liquids, the choice of application varying with the plant species and environmental factors present at the particular locus of application. Thus, the compounds may be formulated as emulsifiable concentrates, as wettable powders, as flowable formulations, as solutions, as dispersions, as suspensions and the like.

The plant regulators of this invention are suitably employed in a number of broad-leafed and grain crops, for example, soybean, lima bean, wheat, rice, corn, sorghum, and cotton, and turf grasses to name a few. In soybean, the compounds of the invention retard senescence and increase yields. In wheat, they retard senescence and exert an antilodging effect. In cotton, the compounds of the invention improve leaf abcission. In turf grasses, the compounds retard growth rate.

A typical formulation may vary widely in concentration of the active ingredient depending on the particular agent used, the additives and carriers used, other active ingredients, and the desired mode of application. With due consideration of these factors, the active ingredient of a typical formulation may, for example, be suitably present at a concentration of from about 0.5% up to about 99.5% by weight of the formulation. Substantially inactive ingredients such as adjuvants, diluents, and carriers may comprise from about 99.5% by weight to as low as about 0.5% by weight of the formulation. Surface active agents, if

employed in the formulation, may be present at various concentrations, suitably in the range of 1 to 30% by weight. Provided below is a general description of exemplary types of formulations which may be employed for dispersion of the plant regulators of the present invention.

Emulsifiable concentrations (EC's) are homogeneous liquid compositions, usually containing the active ingredient dissolved in a liquid carrier. Commonly used liquid carriers include xylene, heavy aromatic naphthas, isophorone, and other nonvolatile or slightly volatile organic solvents. For application, these concentrates are dispersed in water, or other liquid vehicle, forming an emulsion, and are normally applied as a spray to the area to be treated. The concentration of the essential active ingredient in EC's may vary according to the manner in which the composition is to be applied, but, in general, is in the range of 0.5 to 95%, frequently 10 to 80%, by weight of active ingredient, with the remaining 99.5% to 5% being surfactant and liquid carrier.

The following are specific examples of emulsifiable concentrate formulations suitable for use in the present invention:

| Component: | % by Wt. |
|---|---|
| Active ingredient | 53.01 |
| Blend of alkylnaphthalenesulfonate and polyoxyethylene ethers | 6.00 |
| Epoxidized soybean oil | 1.00 |
| Xylene | 39.99 |
| Total | 100.00 |

| | |
|---|---|
| Active ingredient | 10.00 |
| Component: | % by Wt. |
| Blend of alkylnaphthalenesulfonate and polyoxyethylene ethers | 4.00 |
| Xylene | 86.00 |
| Total | 100.00 |

Wettable powders, also useful formulations for plant regulators, are in the form of finely divided particles which disperse readily in water or other liquid vehicles. The wettable powder is ultimately applied to the plant as a dry dust or a dispersion in water or other liquid. Typical carriers for wettable powders include fuller's earth, kaolin clays, silicas, and other highly absorbent or adsorbent inorganic diluents. The concentration of active ingredient in wettable powders is dependent upon physical properties of the active ingredient and the absorbency characteristics of the carrier. Liquids and low melting solids (mp <100°C) are suitably formulated in the concentration range of 5 to 50% by weight, usually from 10 to 30%; high melting solids (mp >100°C) being formulated in the range of 5 to 95% by weight, usually 50 to 85%. An agriculturally acceptable carrier or diluent, frequently including a small amount of a surfactant to facilitate wetting dispersion and suspension, accounts for the balance of the formulation.

The following are specific examples of wettable powder formulations suitable for use in the present invention:

| Component: | % by Wt. |
|---|---|
| Active ingredient | 40.00 |
| Sodium ligninsulfonate | 20.00 |
| Attapulgite clay | 40.00 |
| Total | 100.00 |
| Active ingredient | 90.00 |
| Dioctyl sodium sulfosuccinate | 0.10 |
| Synthetic fine silica | 9.90 |
| Total | 100.00 |

| Component: | % by Wt. |
|---|---|
| Active ingredient | 20.00 |
| Sodium alkylnaphthalenesulfonate | 4.00 |
| Sodium ligninsulfonate | 4.00 |
| Low viscosity methyl cellulose | 3.00 |
| Attapulgite clay | 69.00 |
| Total | 100.00 |
| Active ingredient | 25.00 |
| Base: | 75.00 |
| 96% hydrated aluminum magnesium silicate | |
| 2% powdered sodium lignosulfonate | |
| 2% powdered anionic sodium alkyl-naphthalenesulfonate | |
| Total | 100.00 |

Flowable formulations are similar to EC's except that the active ingredient is suspended in a liquid carrier, generally water. Flowables, like EC's, may include a small amount of a surfactant, and contain active ingredient in the range of 0.5 to 95%, frequently from 10 to 50%, by weight of the composition. For application, flowables may be diluted in water or other liquid vehicle, and are normally applied as a spray to the area to be treated.

The following are specific examples of flowable formulations suitable for use in the present invention:

| Component: | % by Wt. |
|---|---|
| Active ingredient | 46.00 |
| Colloidal magnesium aluminum silicate | 0.40 |
| Sodium alkylnaphthalenesulfonate | 2.00 |
| Paraformaldehyde | 0.10 |
| Water | 41.42 |
| Propylene glycol | 7.50 |
| Acetylinic alcohols | 2.50 |
| Xanthan gum | 0.08 |
| Total | 100.00 |

| Component: | % by Wt. |
|---|---|
| Active ingredient | 45.00 |
| Water | 48.50 |
| Purified smectite clay | 2.00 |
| Xanthan gum | 0.50 |
| Sodium alkylnaphthalenesulfonate | 1.00 |
| Acetylinic alcohols | 3.00 |
| Total | 100.00 |

Typical wetting, dispersing or emulsifying agents used in agricultural formulations include, but are not limited to, the alkyl and alkylaryl sulfonates and sulfates and their sodium salts; alkylaryl polyether alcohols; sulfated higher alcohols; polyethylene oxides; sulfonated animal and vegetable oils; sulfonated petroleum oils; fatty acid esters of polyhydric alcohols and the ethylene oxide addition products of such esters; and the addition product of long-chain mercaptans and ethylene oxide. Many other types of useful surface-active agents are available in commerce. The surface-active agent, when used, normally comprises from 1 to 15% by weight of the composition.

Other useful formulations include simple solutions or suspensions of the active ingredient in a relatively non-volatile solvent such as water, corn oil, kerosene, propylene glycol, or other suitable solvents. This type of formulation is particularly useful for ultra low volume application.

The following illustrate specific suspensions which are suitable for use in the present invention:

| Oil Suspension: | % by Wt. |
|---|---|
| Active ingredient | 25.00 |
| polyoxyethylene sorbitol hexaoleate | 5.00 |
| Highly aliphatic hydrocarbon oil | 70.00 |
| Total | 100.00 |

| Aqueous Suspension: | |
|---|---|
| Active ingredient | 40.00 |
| Polyacrylic acid thickener | 0.30 |
| Dodecylphenol polyethylene glycol ether | 0.50 |
| Disodium phosphate | 1.00 |
| Monosodium phosphate | 0.50 |
| Polyvinyl alcohol | 1.00 |
| Water | 56.70 |
| Total | 100.00 |

The concentration of the compound in use dilution is normally in the range of about 2% to about 0.1%. Many variations of spraying and dusting compositions in the art may be used by substituting or adding a compound of this invention into compositions known or apparent to the art.

The compositions may be formulated and applied with other suitable active ingredients, including nematicides, insecticides, acaricides, fungicides, other plant regulators, herbicides, fertilizers, etc.

In applying the foregoing chemicals, an effective growth regulating amount of the active ingredient must be applied. While the application rate will vary widely depending on the choice of compound, the formulation and mode of application, the plant species being treated and the planting density, a suitable use rate may be in the range of 0.01 to 10 kg/hectare, preferably 0.05 to about 5 kg/hectare.

The compounds of this invention may be prepared by heating an aminopyridine and a substituted phenyl isocyanate in a solvent such as 2-butanone, toluene, or methylene chloride to produce the corresponding N-pyridinylurea. The N-pyridinylurea is then treated with a suitable oxidizing agent, for example metachloroperoxybenzoic acid (MCPBA) in a solvent such as ethanol or ethyl acetate to produce the corresponding (pyridinyl-N-oxide)urea.

The following example illustrates preparation of a compound of this invention.

Example

Synthesis of N′-(2-methyl-4-pyridinyl-N-oxide)-N-phenylurea

N′-(2-methyl-4-pyridinyl)-N-phenylurea and *m*-chloroperoxybenzoic acid produced N′-(2-methyl-4-pyridinyl-N-oxide)-N-phenylurea (m.p. 193—198°C dec.).

NMR (dimethylsulfoxide-$d_6$):
2.38 (s, 3H);
6.90—7.70 (m, 7H);
8.15—8.27 (d, 1H);
8.92 (bs, 1H);
9.20 (bs, 1H).

**Claims**

1. A compound of the formula

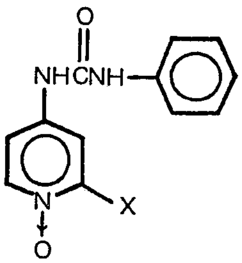

in which X is $C_{1-2}$ alkyl or $C_{1-2}$ alkylthio.

2. A plant growth regulator composition comprising a plant regulating amount of the compound of claim 1 in admixture with an agriculturally acceptable carrier or extender.

3. A method for retarding senescence in soybean plants which comprises applying to the plant a plant regulating amount of the compound of claim 1.

4. A method for increasing yields in soybeans which comprises applying to soybean plants a plant regulating amount of the compound of claim 1.

**Patentansprüche**

1. Eine Verbindung der Formel

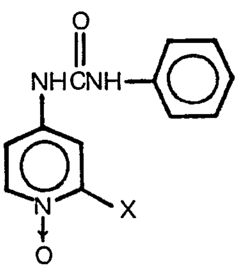

in der X ein $C_{1-2}$-Alkyl- oder ein $C_{1-2}$-Alkylthiorest ist.

2. Pflanzenwachstumsregler-Zusammensetzung, umfassend eine pflanzenregulierende Menge der Verbindung nach Anspruch 1 in Gemisch mit einem in der Landwirtschaft verträglichen Träger oder Streckmittel.

3. Verfahren zur Verzögerung der Alterung von Sojabohnenpflanzen, das das Aufbringen einer pflanzenregulierenden Menge der Verbindung nach Anspruch 1 auf die Pflanze umfaßt.

4. Verfahren zur Steigerung der Ausbeuten von Sojabohnen, das das Aufbringen einer pflanzenregulierenden Menge der Verbindung nach Anspruch 1 auf Sojabohnenpflanzen umfaßt.

**Revendications**

1. Composé de formule:

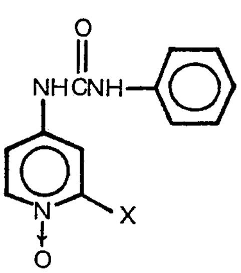

dans laquelle X représente un groupe alkyle en $C_1$—$C_2$ ou un groupe alkylthio en $C_1$—$C_2$.

2. Composition régulatrice de la croissance végétale comprenant une quantité à effet de régulation végétale du composé selon la revendication 1 en mélange avec un véhicule ou une charge acceptable sur le plan agricole.

3. Procédé pour retarder la sénescence des plantes de soja, dans lequel on applique sur la plante, une quantité à effet de régulation végétale du composé selon la revendication 1.

4. Procédé pour accroître les rendements en soja, dans lequel on applique, sur les plans de soja, une quantité à effet de régulation végétale du composé selon la revendication 1.